# EUROPEAN PATENT APPLICATION

(11) **EP 3 572 051 A1**
(43) Date of publication of application: **27.11.2019**
(21) Application number: 18741290.3
(22) Date of filing: 12.01.2018
(51) Int. Cl.: A61F 13/00

(54) **PAD FOR ALLEVIATING AND TREATING PLASMA PROTEIN EXUDATION SKIN DISEASES INCLUDING ATOPIC DISEASES**

(30) Priority: 19.01.2017 KR 20170008987
(71) Applicant: LIPOBIOMED CORPORATION, Gangwon-do 24296 (KR)
(72) Inventor: CHOI, Seong Hyun, Gangwon-do 24610 (KR)
(74) Representative: Berggren Oy, Helsinki & Oulu
(86) International application number: PCT/KR2018/000599
(87) International publication number: WO 2018/135813

(57) **Abstract**

The present invention relates to a pad for treating skin diseases, comprising agar gel and a fiber layer fixed to the inside of the gel, and a manufacturing method therefor. The pad of the present invention inhibits blood vessel exudation symptoms according to vasodilation by maintaining a low temperature of the affected area, reduces an itchy sensation and, at the same time, removes, from the skin, plasma proteins and various plasma-derived substances accumulated in skin tissue, thereby having an effect of alleviating the symptoms of the affected area.

## Description

### [Technical Field]

The present invention relates to a pad which is used in close contact with a lesion to quickly alleviate the symptoms of exudative skin diseases such as atopic dermatitis, eczema and psoriasis.

### [Background Art]

There are several hypotheses and arguments about the etiology of atopic skin diseases. That is, hygiene hypothesis, immune imbalance, excessive consumption of omega-6 polyunsaturated fatty acids and increase of oxidative stress, house dust mite, food allergy and the like have been suggested as causes of the disease, but the causes have not been clarified so far.

Although the etiological cause of atopic skin diseases is not clear, the present invention can provide a method which can alleviate or reduce symptoms based on several biochemical and molecular biological phenomena commonly observed in a symptom-affected area.

The followings are scientific backgrounds on which the present patent is based and theories supporting objects to be sought by the present patent.

### 1. Leakage of plasma protein

Plasma albumin and various plasma proteins with smaller molecular weights are exuded from the blood vessels of the microvascular network distributed below the epidermis and accumulate in the epidermal tissue. Although it is not clear as to whether a phenomenon for vascular exudation of plasma proteins occurs as a result of the occurrence of inflammation or the inflammation occurs due to such exudation phenomenon, the phenomenon for vascular exudation of plasma proteins is one of the common phenomena that are clearly observed. Furthermore, the exudation phenomena that plasma albumin is leaked from the blood vessels is reported in psoriasis, in addition to atopic dermatitis, eczema, seborrheic dermatitis.

It can be expected that plasma albumin and other small-sized plasma proteins that are leaked from the blood vessel wall and accumulated on the epidermal tissue cause the phenomena that they are denatured in the structure, are adhered together, are bound with the epidermal fat molecules, or are agglomerated with fat molecules - because the moisture is relatively insufficient in the epidermal tissue - unlikely when they are present inside the blood vessels. When the phenomena such that proteins are denatured or agglomerated is occurred in the microenvironment within cell-cell of epidermis, they adversely affect the arrangement structure of the surrounding epidermal fat, and eventually, there are high possibility that they damaged the skin barrier function of the epidermal fat preventing the loss of moisture of the epidermis.

In addition, it can be suspected that severe persistent itchy sensation of a patient may be the phenomenon occurred by that the exudate proteins accumulated in the epidermal tissue aggregate into larger sized particles and give physical stimulation to free nerve endings.

### 2. Staphylococcal infection

90% of all atopic patients have Staphylococcal infection. In view of the physiological characteristics of the epidermis, it is necessary to explain why the staphylococcus predominantly grows in the infected area, although there is a possibility that various strains may infect the affected areas of the epidermis due to the continuous exposure of the epidermis to the external environment.

Staphylococcus is a strain that can grow even at high salt concentration. From this fact, it can be inferred that only the small proteins are not leaked from the atopic skin diseases and the similar diseases and accumulated on the epidermal tissue, but that the other ions, such as sodium chloride, calcium, etc., contained in the plasma are leaked and accumulated at the high concentration on the epidermal tissue. In addition, it can be expected that the high concentration of sodium chloride accumulated in the microenvironment of the epidermal tissue accelerate the denaturation and aggregation of the plasma exudate protein molecules, and thereby the intrinsic protective function of the epidermis is further inhibited.

### 3. Moisture evaporation

There is a result that if the moisture routinely evaporated from the epidermis is artificially blocked with water-impermeable membrane, the restoration function of the epidermis is greatly disturbed. The flow of water from the epidermis (its movement from the dermis to the keratinous surface until evaporation) is a very important factor necessary for restoration of the epidermis. Heretofore, there has been a focus on moisturizing and moisture retention in existing methods and attempts to treat or alleviate the symptoms of atopic dermatitis. These means can be seen as results of overlooking the important mechanism that the moisture movement in epidermis is very crucial for epidermal restoration.

### 4. Epidermal temperature

It is natural that epidermal temperature increases when inflammation occurs in skin tissue. However, this elevation of the epidermal temperature further expands the surrounding blood vessels, creating an environment that allows more plasma proteins to exude through the vessel wall. In addition, the increase of the epidermal temperature become a factor for further increasing the evaporation of miosture in the epidermis, and at the same time, the enrichment of various ions contained in the exuded plasma is accelerated. Control of epidermal temperature in atopic skin diseases and similar diseases may be a very important factor in alleviating symptoms, but attempts to control epidermal temperature have not existed until now.

A wet-wrap dressing method to apply steroids and moisturizers for external use has been introduced for the treatment of serious severe atopic symptoms. However, this treatment method is considered as not being popularized. It is concluded that the reason for this is due to the fact that if the moisture evaporation is inhibited, the epidermal restoration is inhibited as mentioned above, the possibility of further vasodilation according to the rise of temperature, and also by this, the results that the exudation of the protein can be increased were not considered.

The present inventors filed a patent application as Korean Patent Application No. 10-2010-0066981 on July 12, 2010, entitled 'Patch for treating and alleviating symptoms of skin diseases associated with blood protein excretion', and a patent application corresponding to Laid-open Patent Publication No. 10-2012-0006315 published on January 18, 2012 describes that the treatment of atopic skin diseases and exudative skin diseases using gel-type pads containing polar ion resin such diethylaminoethyl (DEAE)-cellulose or carboxymethyl (CM)-cellulose, etc.

However, in the process of applying these pads to the skin diseases, the pad with the manner using ion-exchange resin has the closed type attaching it to the epidermis by using Tegaderm™ dressing preventing moisture evaporation, and thus, had problems that the temperature of the epidermis increases when applying it on the skin and itchy sensation is increased when applying it on the skin.

In addition, pure distilled water was used as a medium of a gel to efficiently bind exudate proteins by increasing the charge of the functional groups of the ion-binding resin. Due to the use of distilled water, when it is applied to the skin for a long time (4-5 hours), the phenomenon that the epidermis was blown by water is occurred. Particularly, in the patient with atopic dermatitis, in which the stratum corneum is damaged or keratin is thinned, cases that the degree of blowing of the epidermis by the water is severe, and pain accompanied by this are also observed. After the pad was removed from the affected area, the cases frequently occurred that patients often complained of dryness. It is judged that this dryness is caused by the loss of epidermal fat by exposure to distilled water for a long time. In previous pads using polar resins, there was no assumption that exuded plasma proteins would be present in a state of coalescence inside the epidermal tissue. That is, it may have a limitation in removing plasma-derived exudate proteins that are clustered into large lumps of particles in the cell-cell space of the epidermis.

In addition, when a polar resin is used, it is necessary to thoroughly clean uncoupled free functional groups generated during the production of the resin. Further, since these non-bonding functional groups have a reactive property, it is difficult to confirm that they are completely removed from the resin particles. To increase the efficiency of the polar resins used, it is troublesome to repeatedly wash the resin by using the diluted strong acid (HCl) or diluted base (NaOH) solution to activate the functional groups of the resin. After the cumbersome process of washing the resin, a somewhat difficult process and procedure are required to confirm as to whether the acid or base solution used is completely removed.

### [Disclosure]

### [Technical Problem]

The present invention has been made to solve the above-mentioned problems and made by the above-mentioned necessity, and thus, the object of the present patent is to solve the above-mentioned physiological problems and to provide a new pad which can be manufactured without the cumbersome and difficult manufacturing process.

### [Technical Solution]

In order to achieve the above object, the present invention provides a pad for treating skin diseases, comprising an agar gel and a fiber layer fixed to the inside of the gel. In the present invention, the gel may be theoretically a polyacrylamide gel in addition to an agar gel. However, there is a difficulty in providing an evidence that the harmful components (free acrylamide molecules and radical former, i.e., ammonium persulphate) in a human body are not contained in the gel.

In one embodiment of the present invention, it is preferable that the pad further comprises, not limited thereto, a porous membrane for controlling moisture evaporation on upper part of the fiber layer and/or a net structure for preventing a contact between the cloth and the pad on upper part of the porous membrane.

In one embodiment of the present invention, the pad preferably further comprises a salt of an organic acid and/or an organic acid thereof, and the organic acid may be preferably propionic acid, butyric acid, or acetic acid, but is not limited thereto.

In another embodiment of the present invention, it is preferable that the pad further comprises a phospholipid, and the phospholipid is preferably lecithin, but is not limited thereto.

In another embodiment of the present invention, the fibers are preferably paper, natural fibers or synthetic fibers, and the natural fibers are preferably cotton, silk, or wool, and the synthetic fibers are preferably acrylic, polyester, nylon, or polyethylene, but is not limited thereto.

In one embodiment of the present invention, the skin disease is preferably a disease showing vascular exudation phenomenon of plasma proteins, and the skin disease is more preferably the disease selected from the group consisting of atopic dermatitis, eczema, psoriasis, contact dermatitis, and skin itching, but is not limited thereto.

The present invention also provides a pad to be applied after a mosquito bite, comprising an agar gel and a fiber layer fixed to the inside of the gel.

The present invention also provides a pad for reducing epidermal temperature, comprising an agar gel and a fiber layer fixed to the inside of the gel.

Further, the present invention provides a pad for removing skin wastes or dusts on the surface of a skin, comprising an agar gel and a fiber layer fixed to the inside of the gel.

In addition, the present invention provides a method for manufacturing a pad for treating skin diseases, comprising placing agar in a NaCl solution and sterilizing the solution, and then placing the fiber on the surface of the solution before the solution is hardened and curing it together with the agar so that they are united.

In one embodiment of the present invention, the method is preferable to further add one or more components of organic acid and phospholipid before the solution is hardened, but is not limited thereto.

Hereinafter, the present invention will be described.

The inventors of the present invention consider that one of the important physiological phenomena that exacerbate symptoms in similar skin diseases exhibiting exudation, such as atopic dermatitis, eczema, and psoriasis is the blood vessel exudative phenomena of plasma proteins. It is expected that plasma proteins exuded from the blood vessels cannot be collected again inside the blood vessels and accumulated in the epidermal tissue, and then these proteins can be denatured and aggregated together or aggregated with the surround epidermal fats. That is, it is judged that the barrier function of the epidermis is disturbed and is not properly performed. It is suspected that itching sense that the patients feel is the phenomena occurred by that the exuded proteins are aggregated and cause the physical stimulation on free nerve ends.

It is judged that in addition to the exudation of albumin or smaller plasma proteins through the blood vessels, various ions such as NaCl and Ca²⁺ ions are also exuded and accumulated in the skin tissue. It is also considered that these accumulated ions provide a good microenvironment for the growth of Staphylococcus, disturb the metabolism of skin tissue and also prevent normal restoration of skin tissue.

When inflammation occurs, vasodilation occurs and the temperature of the affected area rises. The increase of epidermal temperature is judged to be factors that further increase exudation phenomena of the blood vessels and plays a role for exacerbating symptoms.

The present invention is to provide the following solution to solve the above-mentioned various problems.

1. Role of lowering the temperature of the epidermis; 2. Role of providing moisture to the epidermis to dilute and reduce the concentration of accumulated sodium chloride and other ions and to facilitate the discharge; 3. Role of loosening the aggregation of exudative proteins accumulated in the epidermis and discharging it out of the epidermis; 4. Role of reducing itching sense; 5. Role of promoting the water molecule of the epidermis to facilitate the restoration of epidermis; 6. Role sought for controlling pathogens by adding antibiotics and germicides, if necessary, and the like. These are objects aimed by the present invention.

The purpose of the present invention is to alleviate and improve symptoms of various skin diseases accompanied by exudation without side effects, rapidly and safely.

The present invention relates to a pad which is applied by closely adhering to the affected area for the purpose of rapidly alleviating and improving symptoms of atopic diseases, eczema, psoriasis, and similar skin diseases accompanied by vascular exudation of various plasma proteins; materials to be contained in the pad; and a method of manufacturing the pad.

### Structure of the pad

The structure of the pad of the present invention has a gel having a fine mesh structure capable of maximizing moisture content as a basic form. It is important that the fine mesh structure of the gel has the structure making smooth migration of macromolecules aggregated with various proteins including water molecules. The components of the gel are not harmful to the human body, and the components that are not degraded by the enzyme secreted by the body and the strain are used. It has 2∼3 mm thickness, and it is possible to use a thicker gel if necessary (in order to extend application time). The bottom of the gel (the surface in contact with the skin) should have a smooth surface that is in close contact with the skin. Agar (agar or agarose) is most suitable as a representative component for forming a gel.

The upper surface of the gel is made of a material which can facilitate the evaporation of water, and rough and tough paper and thin cotton, or fabric woven by cotton fibers woven with many concavo-convex structures on its surface, silk fibers, polyester fibers, acrylic fiber, etc. is used. This is called as a fiber layer. The fiber layer is present on the surface of the gel and is made not to touch the underside that contacts the skin. Such a fiber structure widens the surface area on the gel surface, thereby contributing to helping moisture evaporation while playing a role of enhancing the physical durability of the gel. It aims to protect the gel that is easily damaged by bending, pulling, pressing and the like.

The pad directed by the present invention has the purpose of facilitating the evaporation of moisture at the surface of the gel to lower the temperature of the affected area, but the case was observed that the epidermal temperature is rarely low when the pad is applied, and thus discomfort feeling is complained. In this case, it is possible to additionally selectively use a diaphragm - a membrane formed by puncturing holes at a predetermined interval in thin polyvinyl - capable of controlling the evaporating amount of moisture on the upper surface of the pad. By reducing the moisture-evaporation amount, it is possible to prevent the epidermal temperature from being drastically lowered. The top layer of the pad is provided with a protective cover of a mesh structure (1 mm thick) made of soft plastic, latex or urethane material that does not absorb water to prevent the patient's clothes from getting wet by contacting with it when the pad is applied to the skin. This has the purpose to provide convenience to patients who need to be applied for a long time.

A Surginet™ (a fixed band in the form of a flexible mesh) can be used to bring the pad into contact with the affected area of the patient. When the pad is fixed to the affected portion using the adhesive dressing tape, it is important to expose the surface of the pad as much as possible so as not to disturb moisture evaporation.

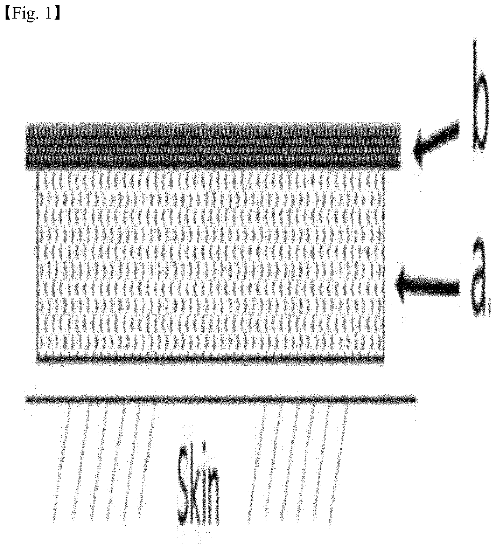

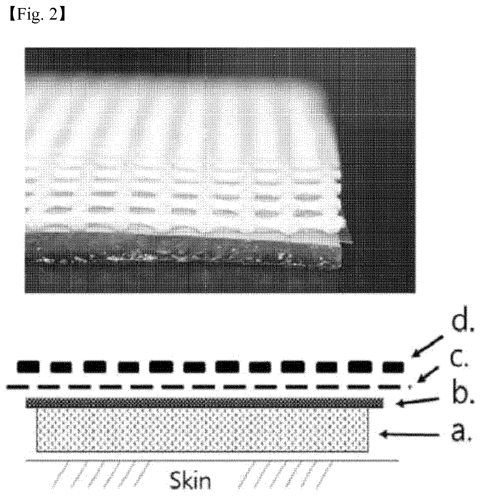

The structure of the pad of the present invention is shown in Figs. 1 and 2. Fig. 1 is a basic structure of the pad of the present invention, and sufficiently accomplished the desired effects of the present invention only by (a) an agar gel, and (b) a fiber layer fixed to the inside of the gel. For reference, 'P-B' (Basic form) in Table 1 means the pad of Fig. 1.

### Function of the pad and description of each active effective ingredient

The gel constituting the pad uses 1.5 to 5% (w/v) agar (1% gel is too runny so that the gel does not maintain its shape when applied to the epidermis, and when it exceeds 5%, it is too hard to adhere to the epidermis and 1.5% gel can also be used even though its properties are somewhat runny), so that it does not contain air bubbles inside the gel. The basic concentration of sodium chloride contained in the gel is made to be 30-95% concentration of physiological saline. [It is difficult to determine the amount of sodium chloride accumulated on the epidermis, but when applying a 2 mm thick gel made of distilled water to the epidermis under the assumption that Sodium chloride in concentration of 3-fold over that of a physiological saline is concentrated on epidermis, the concentration of sodium chloride is 10 times diluted and thus, has a physiological saline concentration of about 30%. That is, since the skin can be swollen by the water due to low concentration of Sodium chloride, the concentration of the gel is to compensate this. There are many cases that patients with atopic dermatitis almost lost their keratin, it is judged that a certain amount of salt concentration by which the epidermis is not swollen can protect the epidermis. In addition, after a pad having a 50% Sodium chloride concentration is applied for about 5 hours, the water is evaporated to half and the concentration inside the gel becomes to a physiological concentration].

A high water content of gel matrix, such as agar, is primarily intended to dilute the proteins, sodium chloride, calcium, various ions and other metabolites accumulated in the skin tissue by supplying moisture to the epidermis.

The high water content of gel matrix maintains the surface temperature of the affected part lower than the surrounding skin tissue by keeping the evaporation of water for a period of time (in the case of 2 mm thick, about 5 hours) on the gel surface. Furthermore, the fine mesh structure of the gel serves to move protein molecules and various ionic molecules accumulated on the skin to the inside of the gel and remove these substances from the epidermis. When the moisture evaporates smoothly on the surface of the gel applied on the skin, the force (pumping action) for pulling up and moving various substances from the epidermis becomes higher (see the experimental results of Examples). The pumping action of the gel makes it possible to remove unwanted molecules accumulated in the epidermis tissue more rapidly. Since the water flow from the basal layer to the keratinous surface through the cell-cell space in the skin tissue is an important factor in the restoration of the epidermis, the moisture pumping action of the pad can play a role to help restoration of the affected area of the specific skin disease.

### Addition of organic acids or short chain fatty acids

The proteins that are exited from the blood vessels and accumulated are exposed to an environment of a relatively dry skin tissue, unlike when they are inside the vessels. Under these conditions, the structure of the protein is likely to be denatured, bound each other, and aggregated. This aggregation is expected to be exacerbated by divalent cations such as calcium accumulated in the surroundings. The exudate proteins, especially plasma albumin, are expected to bind to the unique lipid molecules that the epidermis has, and remain insoluble in water. In other words, it is judged as not being easy to efficiently remove proteins accumulated in the epidermal tissue from the epidermis.

Adding to the matrix of the gel the substances which dissociates the aggregated proteins and which allows the bound protein-fat molecules to be easily separated with each other also facilitates the exudation of the exudate protein through diffusion.
The substance should have a property hindering hydrophobic interaction and ionic bonding at the same time, and it has a small molecular weight so that penetration of the epidermis should be easy. In addition, it should not cause problems in the integrity of the epidermal structure. It should have a property capable of being metabolized without a large effect on the metabolism of epidermal cells, and being decomposed and extinguished.

Propionic acid (a combination of propionic acid/propionate sodium salt) was contained in the gel matrix as a substance satisfying the above conditions.
Butyric acid/butyrate sodium salt combination) or acetic acid (acetic acid/acetate sodium salt combination) having similar structure and properties can be used as an alternative in addition to propionic acid.

The concentration of these organic acids can be used at a level of micromolar concentration as a concentration that does not cause problems in the cell-cell binding structure of the epidermis [the carboxyl group of the organic acid has a property interfering with the action of calcium ions. Calcium ion concentration of epidermis is maintained at nM level. If the concentration is above 100 nM calcium concentration, the epidermal cells is recognized1 as a cleavage signal. It is also related to abnormal proliferation of epidermis. On the other hand, the calcium concentration of blood is 2-3 mM. That is, the mM concentration is required to maintain the structural integrity (related to the tight junction function) of the blood vessel wall, and the nM concentration should be maintained to maintain the epidermal cell stability. Because of this physiological condition, the concentration of µM is selected as the intermediate concentration.]

### Addition of lecithin

There is phospholipid (lecithin) as an amphoteric substance having a hydrophobic property and head group and is highly soluble in water among the epidermal constituents. Lecithin is also a component of epidermal fat, but it can be used as a detergent-like substance or as an emulsifying agent due to its structural property. The present invention uses lecithin by adding it to the gel for the purpose of dissociating the exuded protein from the epidermal fat bound thereto. It can be added to the gel matrix at the milligram (mg/ml) level to improve symptoms of the affected area [The average critical micelle concentration (CMC) value of each of phospholipids constituting lecithin is about 1 mg/ml. When the concentration is lower than that of CMC, the lecithin molecules are difficult to play a detergent-like role, and when the concentration is too high, it becomes difficult to dissolve in an aqueous solution.]

### Other additives.

The pathogen (the infection) which causes the most problematic problem in atopic skin disease is Staphylococcus. 90% of patients have an infection of this species. The control of Staphylococcus can improve the symptoms more quickly, so it can be applied by adding a bactericide and an antibiotic to a gel matrix.

### Effects of the invention

As can be seen from the present invention, the pad of the present invention has roles of lowering the temperature of the epidermis; providing mousture to the epidermis to dilute and reduce the concentration of accumulated Sodium chloride and other ions and to facilitate the discharge; loosening the aggregation of exudative proteins accumulated in the epidermis and discharging it out of the epidermis; reducing itching sense; and promoting the flow of water molecule of the epidermis to facilitate the restoration of epidermal tissue and thus, has the effect alleviating symptoms of various skin diseases including atopic skin disease accompanied by exudative phenomena rapidly and safely, without side effects.

### [Description of Drawings]

Fig. 1 shows a basic form of the pad of the present invention, and Fig. 2 shows a structure manufactured by selectively raising a porous membrane and a net structure in a basic form. In Fig. 1, a. means Agar gel; b. means a fiber layer fixed to the inside of the gel; c. means a porous membrane to control the amount of moisture evaporation (optionally used); d. means a net structure (optionally used) to prevent contact between clothes and pads.
Fig. 3 shows an example of measurement of epidermal temperature using a wireless temperature sensor. A. represents a temperature change of the epidermis when two layers of gauze are placed on the epidermis; B. represents the temperature change of the epidermis when the pad is applied to epidermis; C. represents the temperature change when applied to skin with masked nonwoven fabric soaked with 50% NaCl concentration solution of physiological saline. The arrow indicates when the pad and nonwoven fabric were placed on the epidermis;
Fig. 4 is a graph showing the moisture evaporation rate of the pad of the present invention. After the pad is adhered to a flat heating block set at 32 °C, the weight was measured at a predetermined time interval to determine the amount of moisture evaporation, and based on this, the moisture evaporation rate was calculated. The criterion on the time applied on the skin is defined as the time taking from the NaCl concentration of 50% physiological saline to reach the physiological saline concentration by evaporation of water;
Fig. 5 is a graph showing changes in moisture evaporation rate using moisture evaporation control of the pad of the present invention;

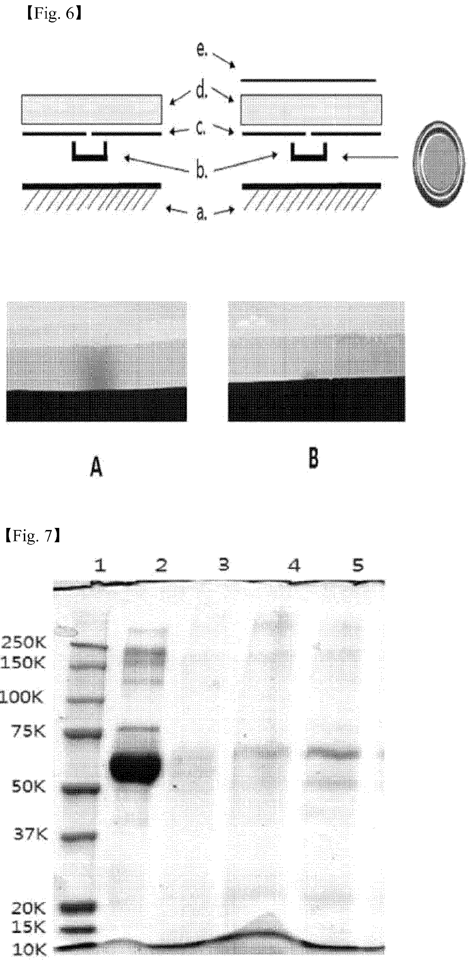

Fig. 6 shows a pumping action of the pad of the present invention. It can be identified that when moisture evaporation occurs at the surface of the pad, a pumping action occurs that moves the material from the bottom to the top of the pad. The right pattern was covered with a wrap on the pad to suppress moisture evaporation, and the left pattern was exposed to the surface to facilitate the evaporation of moisture. When the moisture evaporation is suppressed at the surface of the pad, it can be seen that the bromophenol blue dyeing reagent placed on the lower side of the pad hardly moves (lower right). If the moisture evaporates smoothly on the pad surface, it can be seen that the staining reagent moves up to the surface layer due to the pumping action (lower left). a. A hot plate surface set at 32°C; b. Finn Chamber containing bromophenol blue staining reagent; c. A needle-punched polyvinyl film; d. a pad; e. wrap;
Fig. 7 is a photograph showing the protein pumping action of the pad of the present invention in the affected area of the patient with atopic dermatitis. The pad was applied to the affected area of the patient with atopic dermatitis for 3 hours and 30 minutes, and it is collected, and then the proteins transferred to the pad were analyzed. 10% (w/v) SDS-PAGE gel was used and stained with CBB-R. Lane 1. Molecular weight standard (Bio-Rad); Lane 2. Human serum albumin (Sigma-Aldrich); Lane 3, 4, 5, Protein samples extracted from pads applied to the affected part of patients with atopic dermatitis.
Fig. 8 shows an example for the improvement of symptoms of atopic dermatitis by the pad of the present invention.
Fig. 9 shows an example for the improvement of symptoms of atopic eczema by the pad of the present invention.
Fig. 10 shows an example for the improvement of symptoms of contact dermatitis by the pad of the present invention.
Fig. 11 shows an example for the improvement of symptoms of psoriasis by the pad of the present invention.
Fig. 12 is a photograph showing the result obtained by applying the pad of the present invention to a mosquito bite.
Fig. 13 shows a result of adsorption of the lipstick on the skin by the pad.

### [Mode for Invention]

Hereinafter, the present invention will now be described in more detail by way of nonlimiting examples. Provided that, the following examples are intended to illustrate the present invention and the scope of the present invention is not to be construed as being limited by the following examples.

### Example 1: Preparation of the pad of the present invention

The pad of the present invention was prepared by putting 2% (w/v) agar in a 50% NaCl solution (about 70 mM) of saline, autoclaving (121°C, 15lb/cm², 15 min) and then pouring it into a mold to be a thickness of 2 mm, when cooled at 60-70°C. When the agar solution was maintained in the liquid phase (about 55-70°C) before hardening, fibers (paper, thin cotton, fiber, etc.) were placed on the surface and cured together with the agar gel so as to be united. After the agar gel was completely hardened, the agar film, which occasionally climbed over the fibrous layer and hardened to form, was scraped off with a flat, sharp tool to remove it clearly.

In preparing the pads containing the various compositions described in Table 1 in addition to the basic pads of the present invention, when the gel is in the liquid phase before hardening it (55-70°C), the components to be required (for example, organic acid, lecithin, and the like, as described in Table 1) were added, mixed well and then hardened by pouring them into a mold. Upon mixing the composition required for the gel, care was taken to prevent air bubbles from forming the inside of the gel.

### Experimental example 1: Measurement of epidermal temperature and epidermis cooling effect of the pad

The temperature change of epidermis was monitored using a wireless temperature sensor (SL52T data logger, Signatrol, UK). In order to confirm the effect of the pad on lowering the epidermal temperature, the temperature change was determined for each of cases that temperature sensor was placed on the skin inside the normal forearm, and then covered with two layers of gauze, and that the temperature sensor was placed on the skin, and then the pad (6x8cm, 2mm thickness, 50% NaCl concentration) was placed on the it. In addition, in order to confirm the continuous effect of pad on skin cooling, the temperature changes were compared using nonwoven fabrics (6x8cm)for a mask pack, socked with a salt concentration solution of 50% physiological saline. Experiments were carried out at room temperature, and the mean room temperature at the time of the experiment was 25±1°C and the mean relative humidity was 30%. Each of gauze, pad, and nonwoven fabric soaked in saline, including a temperature sensor, was fixed on the skin using SurgeNet™.

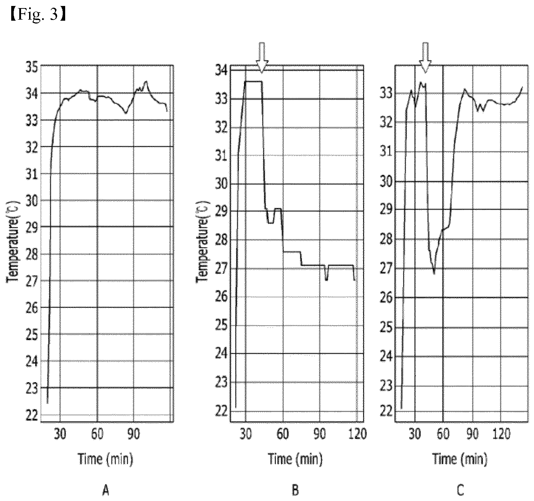

As a result of the experiment, the epidermal temperature of the normal person was 32.5 ± 0.5°C (n=4), and the pad of the present invention was able to lower the epidermal temperature by about 5.8 ± 0.3°C. On the contrary, the wet nonwoven fabric was able to temporarily lower the epidermal temperature, but was found to be non-persistent (Fig. 3).

The temperatures of the normal and affected areas of atopic patients were measured in the same manner as above and compared with the skin temperature of the normal person.

The average temperature of the normal parts of the atopic dermatitis patients was 34.05±0.4°C (n= 2) and the average temperature of affected parts showing inflammation was 34.1± 0.5°C. The epidermal temperature of the affected area with the pad was kept low by an average of 6.25± 0.2°C.

From the above results, the skin temperature of the patient with atopic skin disease was about 1.5°C higher than that of the normal person, and the temperature of the affected part was finely higher than the other normal parts of the patient. It can be inferred that the atopic skin disease is associated with vasodilatory factors.

When applying the pad of the present invention, it was observed that the skin temperature was lowered at the affected area of the atopic patient as compared with the normal skin. This reason can be explained by the phenomenon occurred by that the epidermal temperature is relatively high and thus the evaporation of water on the pad surface is increase.

### Experimental example 2: The duration for lowering the epidermal temperature by the pad of the present invention

A pad having 2mm thickness was closely attached to the surface of a heating block flat plate set at 32°C (11.3 x 6.6 cm custom-made, Dry Bath, Thermo Scientific™, USA), and the weight of the pads was measured at constant intervals to calculate the amount of moisture evaporated. The gel of the pad was prepared so as to contain 50% NaCl concentration of physiological saline, and based on this, the time point at which 50% of the moisture of the pad is evaporated to be higher to the physiological salt concentration is set as the epidermis application time. As a result of the experiment, the application time of the pad was measured to be about 4 hours and 30 minutes. It is expected that the amount of moisture evaporation will be further increased because the heat generation in the affected area of the patient is slightly higher than that of the normal skin. That is, when using pads having the same thickness, it is possible to apply a relatively short application time or to extend the application time by increasing the thickness of the pad.

### Experimental example 3: Adjustment of the pad of the present invention for lowering the epidermal temperature

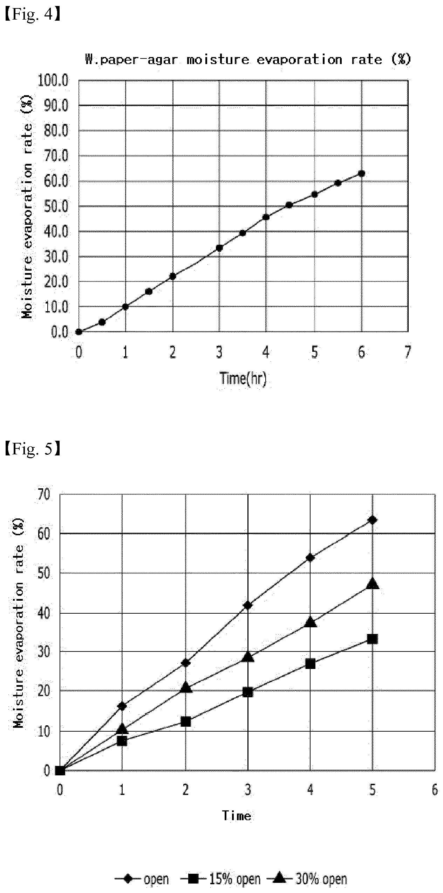

When the pad is applied to the epidermis, the effect is shown that the epidermal temperature of the applied area is lowered by about 6°C under the environmental conditions fixed given in the laboratory. Occasionally, a user may feel cold due to a rarely lowered temperature difference. To alleviate this discomfort, it is necessary to control the degree of lowering of the epidermal temperature by adjusting the moisture evaporation amount of the pad. It is possible to control the lowering of the epidemal temperature by attaching a polyvinyl film perforated at constant intervals to the surface of the pad and controlling the moisture evaporation. When moisture evaporation was measured at the surface of the hot plate set at 32°C, the evaporation rate of moisture was reduced by 64% when the pads were covered with the vinyl film perforated in the area of 30%, and when the pads were covered with the vinyl film perforated in the area of 15%, the moisture evaporation was reduced by 52% (Fig. 5). When measuring the effect was shown that the epidermal temperature of the perforated vinyl membrane in normal skin, the effect of lowering the epidermal temperature was reduced by 3.1 ± 0.1°C for case covered with vinyl film perforated in the area of 15%, and was reduced by 4.4 ± 0.1°C for the case covered with the vinyl film perforated in the area of 30%. When comparing them with the case that the pads that did not cover the surface reduced the epidermal temperature by 6.25 ± 0.2°C, they have the epidermal temperature-reduction effects of about 50% and about 70%, respectively.

Such a temperature control method can be selectively used as a method for reducing the inconvenience caused by the patient's cold. In addition, it can be used as a method for delaying the evaporation of moisture and extending the application time of the epidermis of the pad.

By controlling the moisture evaporation of the pad surface, the effect of lowering the skin temperature of the pad can be controlled. The pad surface was covered with a 15% and 30% perforated vinyl film, respectively, to control the moisture evaporation. The moisture evaporation was reduced by 52% when 15% of the surface of the pad was exposed, and moisture evaporation was reduced by 64% when 30% of the pad surface was exposed. The effect of lowering the epidermal temperature was lowered to about 50% and 70%, respectively, as compared with the pad of which entire surface was exposed.

### Experimental example 4: Pumping action of the pad of the present invention by moisture evaporation

When moisture evaporates from the surface of the pad, a phenomenon occurs in which the materials are rapidly moved from the bottom surface of the pad toward the top surface. Pumping action is an important mechanism used to remove plasma proteins, various ions and organic materials accumulated in atopic skin diseases and various exudative skin diseases from the epidermis. In this experiment, the following experiment was designed to demonstrate the pumping action of the pad.

A Finn Chamber (diameter 11 mm) for Filter Paper Disc was placed on a heating block set at 32°C and fully filled with 0.1% (w/v) bromophenol blue (BPB, molecular weight 669.9) solution. After covering the chamber with a wrap, a hole was made with a needle having a constant thickness in the center of the chamber, and the pad was brought into contact with it immediately above.
The movement of the BPB staining reagent was observed while the top surface of the pad was normally exposed to air and the upper surface of the pad was covered with a wrap (Fig. 6). In this experiment, 6 mm thick pads were made and used to visualize the movement of BPB in the gel layer of the pad. The observation time was 90 minutes.

As moisture evaporates from the surface of the pad, a force acts to pull the material up from the bottom of the pad to the upper surface of the pad. This pumping action of the pad can be applied to remove plasma proteins accumulated from the epidermis, various ions derived from plasma and other organic substances. From this result, it is important to expose the upper surface of the pad to the outside to keep the moisture evaporation smoothly, when the pad is applied to the skin.

### Experimental example 5: The pumping function of the protein of the present invention in the affected area of the patient with atopy

After the pad was applied to the affected area of the patient with atopic dermatitis for 3.5 hours, it was collected to identify the proteins transferred into the gel of the pad. The pad which was applied to the patient for 3 hours and 30 minutes and collected were diced with a razor and then incubated in a 4x SDS-PAGE sample buffer for 1 hour. The crushed gel fragments and SDS-sample buffer were centrifuged (at 500 g for 30 min) using 0.45 micrometer, Spin-X centrifuge tubes (Coster®, Corning®). The collected samples were developed on a 10% (w/v) SDS-PAGE gel. The developed proteins were visualized by Coomassie Brilliant Blue-R250 staining method. As a result of the experiment, it was confirmed that plasma albumin (67 kDa) and small proteins were migrated into the pad. It shows the result that pads have an effect of lowering the epidermal temperature and at the same time have the effect of eliminating the proteins accumulated in the skin tissue by exuding from the blood vessels, thereby helping to improve the symptom of atopic dermatitis and the similar exudative skin diseases (Fig. 7).

### Expermental example 6: Major components of the pad and test verifying the effect for improving the symptoms

The pads aimed by the present invention have effects of shrinking blood vessels around the affected area by constantly lowering the epidermal temperature by only a unique basic structure and removing exuded proteins accumulated on the epidermis to improve symptoms. However, if the exuded proteins are more effectively removed and simultaneously the itchy sensation is reduced, the effect for improving the symptoms can be made more rapidly, and thus, it has been attempted to improve the symptom improvement effect by adding organic acid and lecithin to the composition of the pad. The organic acids used in the present invention were used for the purpose of hindering the hydrophobic binding and the ionic bonding of the organic acid, and lecithin (total phospholipids extracted from animals or plants) was used as a material with a detergent-like property.

As the used organic acids, acetic acid and butyric acid were used by adding them to the pads in the same concentration, based on propionic acid, and they were compared for the effects improving symptoms.

The lecithin used in this experiment was palm lecithin and lecithin extracted from pig lung tissue, respectively. Palm lecithin was extracted from crude palm oil by the method (Pardun H. (1988), Pflanzenlecithine. Gewinnung, Eigenschaften, Verarbeitung und Anwendung pflanzlicher Phosphatidpraparate. Ziolkowsky, Augsburg ISBN 3-87846-128-3). Lecithin extracted from lung tissue of pigs was extracted and purified by the method described by Moon, et al. (Moon J. S., Jeon B. S., Yoon B. I., Choi S. H. and Lim C. J. (2012) Mol. Biol. Rep. 39:4237-4247).

In order to investigate the efficacy and effect of the pad of the basic form and the pad containing various compositions of the present invention, the experiment candidates were selected from patients suffering from atopic dermatitis, atopic eczema, and contact dermatitis. Participants were patients diagnosed with each disease at local or general hospitals. Patients were told about the safety of the ingredients in the pads, but they were made not to notice as to which ingredients of pads were applied. The application time of each pad was 3 hours and 30 minutes, and after the pad was removed from the affected area, a photograph was taken after the lapse of 20 minutes to compare the degree of improvement of the skin.

In order to assess efficacy and effect, two items were made to be evaluated by the patients themselves. 1. How much did the itchy sensation decrease, 2. What is the degree to which the skin is improved by the patient; With respect to these two items, it was evaluated as - 3 points with respect to that the state became worse and +3 points with respect to that the effect is better, based on 0 point with respect to that no effect is improved. In addition to the two items that the patients themselves evaluated, three researchers compared the photographs of patients before and after the treatment of the patients, and then, the inflammation reduction effect was evaluated as a score and the average value was reflected in the efficacy and effect evaluation.

Table 1 shows the pads formed to observe the symptom improvement effect.

**[Table 1]**

| Pad Name | Composition of the components |
|---|---|
| P-FB | Non-woven fabric (soaked with 50% physiological saline concentration) |
| P-B | Pad (Basic form) |
| P-BP | Pad + 10microM propionic acid/propionate sodium salt (p5.8) |
| P-BA | Pad + 10microM acetic acid/acetate sodium salt (pH5.8) |
| P-BB | Pad + 10microM butyric acid/butyrate sodium salt (pH5.8) |
| P-BPLL | Pad + 10microM propionic acid/propionate sodium salt (p5.8) + lecithin isolated from pig lung tissue |
| P-BPPL | Pad + 10microM propionic acid/propionate(p5.8) + lecithin isolated from Palm |

Table 1 shows the types of pads used for the efficacy and effect test.

### Results obtained after applying atopic dermatitis

**[Table 2]**

| Kinds of the pad | Reduction of itchy sensation (Self-assessment) | Improvement for symptoms of the skin (Self-assessment) | Improvement of dermatitis (Assessment by photograph result) | Numbers of patients |
|---|---|---|---|---|
| P-F | Not assessable | Not assessable | 0.5 | 2 |
| P-B | 1.5 | 1.5 | 0.58 | 3 |
| P-BP | 1.7 | 1.7 | 0.92 | 13 |
| P-BA | 1.0 | 1.0 | 0.5 | 2 |
| P-BB | 1.6 | 1.6 | 0.87 | 2 |
| P-BPLL | 1.7 | 1.7 | 1.15 | 5 |
| P-BPPL | 2.0 | 2.0 | 1.14 | 6 |

Table 2 shows the results of the efficacy and effect assessment of Pad when applied to patients with atopic dermatitis.

In order to investigate the effect of each of the animal and vegetable lecithin added to the pad of the present invention on efficacy, it was applied to patients having atopic eczema. Evaluation was performed in the same manner as patients with atopic dermatitis. The results are as follows (Table 3).

**[Table 3]**

| Kinds of the pad | Reduction of itchy sensation (Self-assessment) | Improvement for symptoms of the skin (Self-assessment) | Improvement of dermatitis (Assessment by photo result) | Numbers of patients |
|---|---|---|---|---|
| P-BP | 2.3 | 1.8 | 0.75 | 4 |
| P-BPLL | 2.5 | 1.5 | 0.8 | 2 |
| p-BPPL | 2.0 | 1.5 | 0.17 | 3 |

Table 3 shows the results of the efficacy and effect evaluation when the pad was applied to patients with atopic eczema.

In order to investigate the effect of added additional lecithin in addition to the organic acids added to the basic pad, the effects were evaluated by applying it to patients with contact dermatitis. The evaluation method is the same as that applied to patients with atopic dermatitis and atopic eczema, as mentioned above.

**[Table 4]**

| Kinds of the pad | Reduction of itchy sensation (Self-assessment) | Improvement for symptoms of the skin (Self-assessment) | Improvement of dermatitis (Assessment by photo result) | Numbers of patients |
|---|---|---|---|---|
| P-BP | 1.0 | 1.5 | 0.5 | 2 |
| P-BPLL | 1.5 | 1 | 0.9 | 2 |
| p-BPPL | 1.0 | 1.5 | 0.8 | 2 |

Table 4 shows the results of the efficacy evaluation when pad was applied to patients with contact dermatitis.

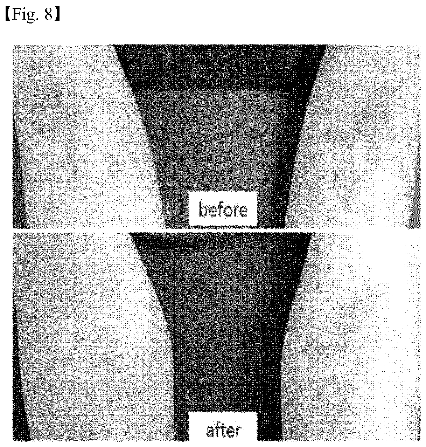

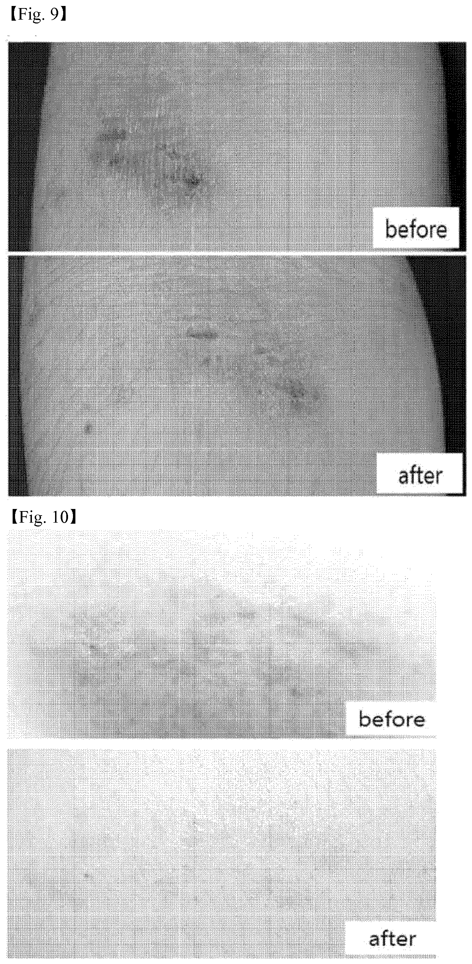

When the pad with various compositions were applied to patients with atopic dermatitis, eczema, and contact dermatitis, the pads showed symptomatic improvement to the degree that can be confirmed by naked eyes even by one application (3 hours 30 minutes) (Examples of symptom improvements for each disease: Figs 8, 9, 10 and 11). Patients who participated in the study had varying severity of symptoms and thus could not be evaluated by an absolute comparative assessment, but it is clear that the pad reduces itchy sensation, improves skin symptoms and reduces skin redness. In particular, the addition of organic acids to the pad makes the reduction of itchy sensation and the improvement of symptoms more clear, and when organic acids and lecithin are added together, the assessment score for symptom improvement is higher. Among the organic acids used as the composition in the pad of the present invention, each of propionic acid and butyric acid can be evaluated as being slightly better than the effect of acetic acid on symptom improvement. The animal lecithin (phospholipid extracted from pig lung tissue) and the vegetable lecithin (phospholipid extracted from crude palm oil), as further added, exhibited symptom improvement effect better than that which the organic acid was used alone, in relation to the effect on the improvement of symptoms. When comparing the effects of only both of animal lecithin and vegetable lecithin, it may be judged that animal lecithin is slightly better, but since the difference in the scores is very small and the conditions of patients applied are different, it cannot be absolutely compared the superiority of both of lecithins. For reference, various patients were asked about the time when the itchy sensation was significantly reduced upon applying the pad, but they were different on the time when each of them feels. However, they exhibited a significant difference according to diseases, and for contact dermatitis (n = 5), the itchy sensation was decreased within 5 minutes after applying the pad, and for atopic dermatitis (n = 29) and atopic eczema (n = 9), it was decreased within 90 minutes and forty seven minutes, respectively.

### Application of the pad of the present invention (psoriasis)

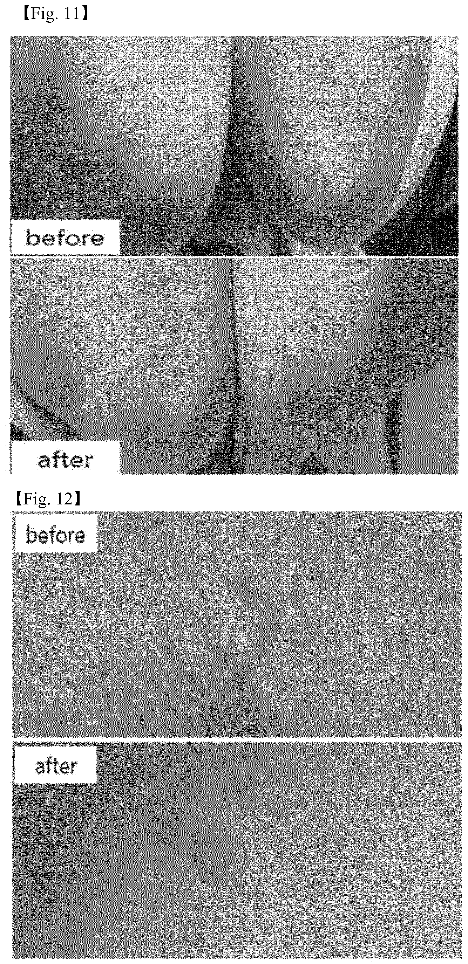

The improvement effect was confirmed by applying the P-BPPL Pad described above to the epidermis of the patient with psoriasis. After applying the relevant pad for 4 hours, the photographs were taken to confirm the improvement of symptoms. It was clearly observed that the background color of the affected area was decreased, and the texture of the keratin was changed. (Fig. 11).

### Application of the pad of the present invention (mosquito bite)

When the mosquito bites, itchy sensation and local warmth are produced together with vasodilatation. Since the pad of the present invention has functions for vasoconstriction, cooling the affected areas, and reduction of itchy sensation, the effect is observed by applying it to the skin bitten by the mosquito. There are three observation opportunities in total. The pad used at this time was pad containing propionic acid in concentration of 20 times or more (20 micro-M propionic acid/ propionate, pH 5.8) than that of Pad P-BP (containing 1 micro-M propionic acid/ propionate) described above. After application of the pad, it was observed that the time taken until itchy sensation was disappeared was 15 minutes on average, and the time taken until a wheal generated by the mosquito bite was gone down was about 45 minutes (Fig. 12).

### Results of Other observations

When the concentration of organic acids is increased, the time until the reduction of the itchy sensation felt by patients is accelerated, however, in some cases, the condition of the affected area may be deteriorated. In particular, when the pad containing propionic acid having the concentration of 20 micro-M was applied of the present invention for 1 hour, there was a case that the affected area was swollen and oozing became more severe. This is presumed to be the result that propionic acid was excessively transferred to the skin under the condition that the skin barrier of the patient with atopic dermatitis was damaged.

The calcium ion concentration required for stabilization of the epidermal structure is nM (nano-M) level, but there is a possibility that the highly transferred propionic acid may have been interfered with the intrinsic function of calcium. On the other hand, in the skin of normal person, it was applied such that the concentration of propionic acid in the pad was increased to 60 micro-M concentration, but no negative effect was observed. On the other hand, patients suffering from atopic dermatitis for a long time and thus having the thickened keratin (lichenification) did not show any skin abnormal reactions even though the pad containing 40micro-M of propionic acid was used.

1 mg/ml lecithin was used in the efficacy and effect test using lecithin, but no abnormal reaction was observed even though pad containing 80 mg/ml phospholipid was applied to an atopic patient with the damaged keratin. It is assumed to be the result due to that since the phospholipid molecules contained in lecithin has larger molecular weight than that of organic acid molecules, the amounts of phospholipid molecules delivered to the inside of the skin are relatively small and most of the molecules are moved to the upper surface of the gel by pumping action of the pad.

### Application as cosmetics

In connection with the pad of the present invention, in the detailed description of the present invention, it has demonstrated the adsorption of a dyeing reagent with a molecular weight of 700 Da and shown the adsorption of proteins in the treatment of atopic patients. That is, it can be inferred that such adsorption function of the pad can exhibit a cleansing effect removing the wastes of the skin by using the pad as cosmetics.

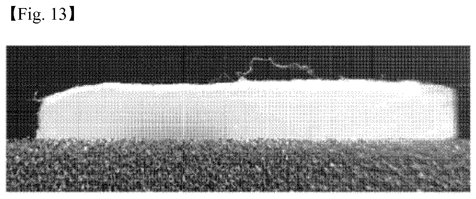

Fig. 13 shows the results that the pad adsorbs the lipstick applied on the epidermis. Skin application time is one hour.

As can be seen in the photograph of Fig. 13, it can be confirmed that the lipstick did not spread sideways but was pulled upwards with a boundary. These adsorption functions play a role in removing cosmetic ingredients that may remain on the epidermis by the use of women using various cosmetics. Especially, it can be applied for the purpose of 'removing the fine dust attached to the epidermis' which is popular nowadays.

## Claims

1. A pad for treating the skin diseases, comprising an agar gel and a fiber layer fixed to the inside of the gel.

2. The pad according to claim 1, wherein the pad further comprises a porous membrane for regulating amounts of the moisture evaporation on the upper surface of the fiber layer.

3. The pad according to claim 2, wherein the pad further comprises a net structure for preventing contact between the clothes and the pad on the upper surface of the porous membrane.

4. The pad according to claim 1, **characterized in that** the pad further comprises at least one of an organic acid and a salt thereof.

5. The pad for treating skin diseases according to claim 4, **characterized in that** the organic acid is propionic acid, butyric acid or acetic acid.

6. The pad for treating skin diseases according to claim 1, **characterized in that** the pad further comprises phospholipids.

7. The pad for treating skin diseases according to claim 6, **characterized in that** the phospholipid is lecithin.

8. The pad for treating skin diseases according to claim 1, **characterized in that** the fiber is paper, natural fiber or synthetic fiber.

9. The pad for treating skin diseases according to any one of claims 1 to 8, **characterized in that** the skin disease is a disease showing vascular exudation of plasma proteins.

10. The pad for treating skin diseases according to any one of claims 1 to 8, **characterized in that** the skin disease is a disease selected from the group consisting of atopic dermatitis, eczema, psoriasis, contact dermatitis, and skin pruritus.

11. A pad for applying after mosquito bite, which comprises an agar gel and a fiber layer fixed to the inside of the gel.

12. A pad for decreasing an epidermal temperature, which comprises an agar gel and a fiber layer fixed to the inside of the gel.

13. A pad for removing wastes from skin or removing dust adhering to the skin surface, which comprises an agar gel and a fiber layer fixed to the inside of the gel.

14. A method for preparing a pad for treating skin diseases, comprising steps for placing agar in NaCl solution and sterilizing it; placing fibers on the surface of the solution before the solution is hardened and then curing them to be united with agar.

15. The method for preparing the pad for treating skin diseases according to claim 14, **characterized in that** it further comprises adding at least one of an organic acid and a phospholipid before the solution hardens.

16. The method for preparing the pad for treating skin diseases according to claim 14, **characterized in that** the fiber is paper, natural fiber or synthetic fiber.

17. The method for preparing the pad for treating skin diseases according to any one of claims 14 to 16, **characterized in that** the skin disease is a disease showing vascular exudation of plasma proteins.
